# EUROPÄISCHE PATENTANMELDUNG

(11) **EP 0 863 129 A1**
(43) Veröffentlichungstag der Anmeldung: **09.09.1998**
(21) Anmeldenummer: 98100594.5
(22) Anmeldetag: 15.01.1998
(51) Int. Cl.: C07C 209/36, C07C 209/10, C07C 269/04

(54) **Verfahren zur Herstellung von N-Carboxyalkyl-3-fluor-4-dialkylaminoanilinen**

(30) Priorität: 07.03.1997 DE 19709443
(71) Anmelder: Clariant GmbH, 65929 Frankfurt am Main (DE)
(72) Erfinder: Schach, Thomas, Dr., 64579 Gernsheim (DE); Schubert, Hans, Dr., 65779 Kelkheim (DE)

(57) **Zusammenfassung**

Verfahren zur Herstellung von N-Carboxyalkyl-3-fluor-4-dialkylaminoanilinen, dadurch gekennzeichnet, daß man in einem ersten Schritt 2-Chlor-4,5-difluornitrobenzol der Formel (I) 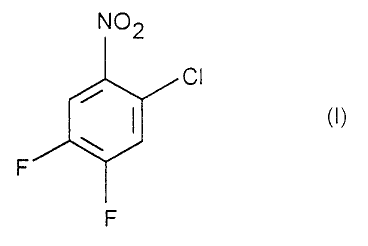 mit einem sekundären Amin der Formel (II) HNR¹R², worin R¹ und R² unabhängig voneinander gleich oder verschieden sind und für einen Alkylrest mit 1 bis 10 Kohlenstoffatomen stehen oder zusammen mit dem N-Atom, an dem sie stehen, einen Ring mit 5 bis 7 Gliedern bilden, in Gegenwart eines wasserlöslichen Amins, das bei Reaktionstemperatur und Aufarbeitungstemperatur flüssige und im Reaktionsgemisch lösliche Hydrofluoride und Hydrochloride bildet, in Anwesenheit oder Abwesenheit eines Lösungsmittels bei -10 bis 120°C umsetzt, in einem zweiten Schritt das 2-Chlor-4-dialkylamino-5-fluornitrobenzol enthaltende Reaktionsgemisch in Anwesenheit des wasserlöslichen Amins in Gegenwart eines Edelmetallkatalysators mit Wasserstoff bei 30 bis 150°C und 1 bis 350 bar reduziert und in einem dritten Schritt das 3-Fluor-4-dialkylaminoanilin enthaltende Reaktionsgemisch in Anwesenheit des wasserlöslichen Amins mit einem Chlorameisensäureester der Formel (IV) ClCO₂R³, worin R³ für einen Alkylrest mit 1 bis 10 Kohlenstoffatomen oder Aralkylrest mit 7 bis 20 Kohlenstoffatomen steht, bei 0 bis 100°C umsetzt.

## Beschreibung

Die vorliegende Erfindung betrifft ein gegenüber dem Stand der Technik verbessertes Verfahren zur Herstellung von N-Carboxyalkyl-3-fluor-4-dialkylaminoanilinen.

N-Carboxyalkyl-3-fluor-4-dialkylaminoaniline spielen als Zwischenprodukte bei der Herstellung von Pharmazeutika (WO-95/25106) bedeutende Rolle.

Wie aus der WO 95/25106 hervorgeht, dient N-Carboxybenzyl-3-fluor-4-piperidinoanilin als Vorprodukt zur Herstellung Oxazolidinonderivaten und diese Derivate enthaltenden pharmazeutischen Zusammensetzungen.

Zur Herstellung des N-Carboxybenzyl-3-fluor-4-piperidinoanilins (Beispiel 1 der WO 95/25106) setzt man zunächst in einem ersten Schritt 3,4-Difluornitrobenzol in Anwesenheit von Diisopropylethylamin mit Piperidin in Ethylacetat um, fügt zu der Reaktionslösung Wasser zu, trennt die Ethylacetatphase ab, wäscht diese mit Wasser und Salzlauge und trocknet über wasserfreiem Natriumsulfat. Anschließend dampft man das Lösungsmittel ab und erhält die Nitroverbindung (3-Fluor-4-piperidinonitrobenzol). Man löst die Nitroverbindung in Ethylacetat und hydriert in Gegenwart eines Palladiumkatalysators, filtriert den Katalysator ab, dampft unter Vakuum ein und erhält das entsprechende Amin (3-Fluor-4-piperidino-anilin). In einem dritten Schritt läßt man das in Tetrahydrofuran gelöste Amin mit Natriumhydrogencarbonat und Chlorameisensäureester reagieren und fügt nach Abschluß der Reaktion Wasser zu, trennt die Tetrahydrofuranlösung ab, wäscht sie mit Wasser und Salzlauge und trocknet über wasserfreiem Natriumsulfat. Nach Abdampfen des Lösungsmittels wird das Produkt mittels Säulenchromatographie gereinigt.

Das in der WO 95/25106 beschriebene Verfahren zur Herstellung von N-Carboxybenzyl-3-fluor-4-piperidinoanilin weist mehrere Nachteile auf. Zum einen kann man lediglich ein einziges Edukt, nämlich 3,4-Difluornitrobenzol, verwenden und zum anderen handelt es sich bei dem 3,4-Difluornitrobenzol um ein recht kostspieliges Produkt, das sich nur über eine sehr aufwendige, mehrstufige Synthese herstellen läßt. Weitere Nachteile bestehen darin, daß das Verfahren sehr viele Einzelschritte erfordert und ein jedes Zwischenprodukt isoliert wird. Darüber hinaus erfordern die einzelnen Reaktionsschritte einen nicht unerheblichen Zeitaufwand, der für die erste Stufe 2 Tage und für die beiden anderen Stufen jeweils 14 Stunden beträgt.

Im Hinblick auf die vorangegangenen Darlegungen besteht ein Bedarf, ein Verfahren zur Herstellung von N-Carboxyalkyl-3-fluor-4-dialkylaminonanilinen bereitzustellen, das die genannten Nachteile vermeidet und sich mit einem vertretbarem Arbeits- und Zeitaufwand realisieren läßt. Darüber hinaus soll sich dieses Verfahren nicht auf die Herstellung von N-Carboxyalkyl-3-fluor-4-piperidinoanilinen beschränken, sondern weitere Verbindungen aus dieser Stoffgruppe zugänglich machen.

Gelöst wird diese Aufgabe durch ein Verfahren zur Herstellung von N-Carboxyalkyl-3-fluor-4-dialkylaminoanilinen. Es ist dadurch gekennzeichnet, daß man in einem ersten Schritt 2-Chlor-4,5-difluornitrobenzol der Formel (I) 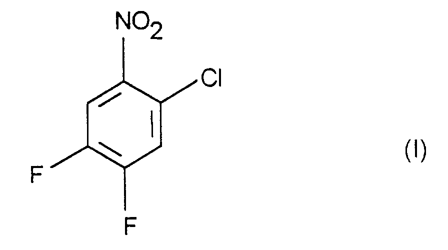 mit einem sekundären Amin der Formel (II) HNR¹R², worin R¹ und R² unabhängig voneinander gleich oder verschieden sind und für einen Alkylrest mit 1 bis 10 Kohlenstoffatomen stehen oder zusammen mit dem N-Atom, an dem sie stehen, einen Ring mit 5 bis 7 Gliedern bilden, in Gegenwart eines wasserlöslichen Amins, das bei Reaktionstemperatur und Aufarbeitungstemperatur flüssige oder im Reaktionsgemisch lösliche Hydrofluoride und Hydrochloride bildet, in Anwesenheit oder Abwesenheit eines Lösungsmittels bei -10 bis 120°C umsetzt, in einem zweiten Schritt das 2-Chlor-4-dialkylamino-5-fluornitrobenzol enthaltende Reaktionsgemisch in Anwesenheit des wasserlöslichen Amins in Gegenwart eines Edelmetallkatalysators mit Wasserstoff bei 30 bis 150°C und 1 bis 350 bar reduziert und in einem dritten Schritt das 3-Fluor-4-dialkylaminoanilin enthaltende Reaktionsgemisch in Anwesenheit des wasserlöslichen Amins mit einem Chlorameisensäureester der Formel (IV) ClCO₂R³, worin R³ für einen Alkylrest mit 1 bis 10 Kohlenstoffatomen oder Aralkylrest mit 7 bis 20 Kohlenstoffatomen steht, bei 0 bis 100°C umsetzt.

Die Synthesestrategie des erfindungsgemäßen Verfahrens macht sich den Umstand zunutze, daß ausgehend von 2-Chlor-4,5-difluornitrobenzol, alle Schritte in Gegenwart einer basischen Verbindung durchgeführt werden können. Wählt man eine geeignete basische Verbindung, so ist es möglich, das Verfahren in allen Schritten in Gegenwart ein und derselben basischen Verbindung durchzuführen. Verwendet man in allen Schritten der Synthese ein und derselbe basische Verbindung, nämlich ein wasserlösliches Amin, das unter den Bedingungen der Reaktion und Aufarbeitung, insbesondere bei Reaktionstemperatur und Aufarbeitungstemperatur flüssige oder im Reaktionsgemisch lösliche Hydrofluoride und Hydrochloride bildet, so läßt sich die Umsetzung sowohl in mehreren separaten Schritten als auch besonders einfach als Eintopfreaktion durchführen. Desweiteren wird insbesondere das wesentlich leichter als das im Verfahren gemäß WO 95/25106 verwendete 3,4-Difluorbenzol herzustellende 2-Chlor-4,5-difluornitrobenzol als Synthesebaustein verwendet, da das nicht benötigte Chloratom während der Synthese selektiv abgespalten werden kann. Der Vollständigkeit halber sei erwähnt, daß sich 2,4-Dichlor-5-fluornitrobenzol auf einfache Weise durch Nitrierung von 2,4-Dichlorfluorbenzol herstellen läßt.

Ein Vorteil des erfindungsgemäßen Verfahrens besteht ferner darin, daß man ohne Isolierung der Zwischenprodukte die gewünschten N-Carboxyalkyl-3-fluor-4-dialkylaminoaniline herstellen kann. Man kann zwar die entsprechenden Zwischenprodukte isolieren, um sie anschließend weiterzuverarbeiten. Man kann aber auch auf eine aufwendige Zwischenisolierung verzichten und das gesamte Verfahren in einer Eintopfreaktion durchführen. Aus technischer Sicht kann es sinnvoll sein, die Eintopfsynthese in mehreren Reaktoren durchzuführen oder den verwendeten Katalysator im Laufe des Verfahrens durch Filtration zu entfernen. Vorteilhafterweise wird das Verfahren über alle Syntheseschritte ohne jegliche Reinigung und/oder Isolierung von Zwischenprodukten, lediglich durch Abtrennen von Hilfsstoffen, bis zur Isolierung des gewünschten N-Carboxyalkyl-3-fluor-4-dialkylaminoanilines durchgeführt.

Das erfindungsgemäße Verfahren zur Herstellung von N-Carboxyalkyl-3-fluor-4-dialkylaminoanilinen ist durch das nachfolgende Reaktionsschema in vereinfachter Form wiedergegeben.

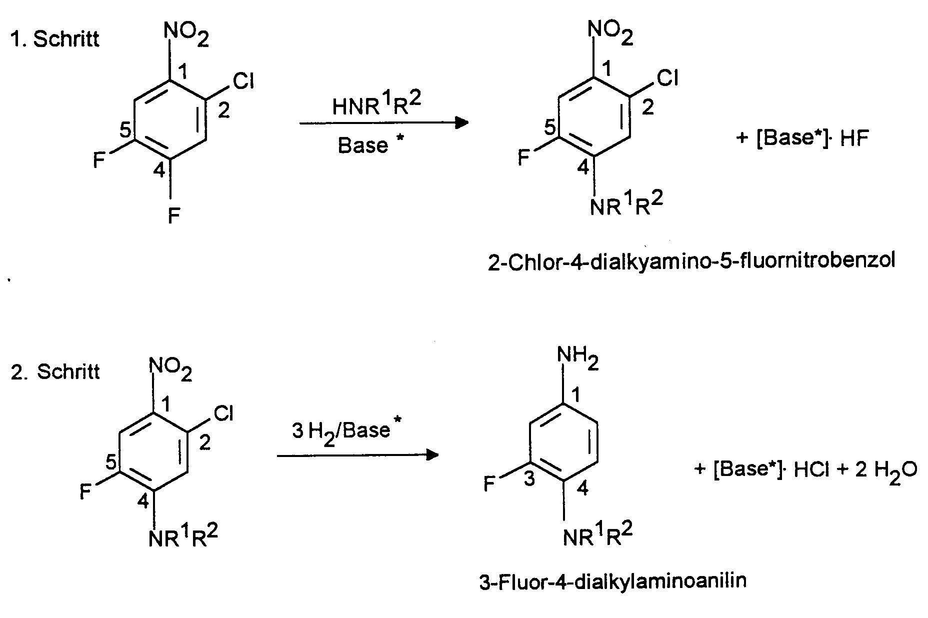

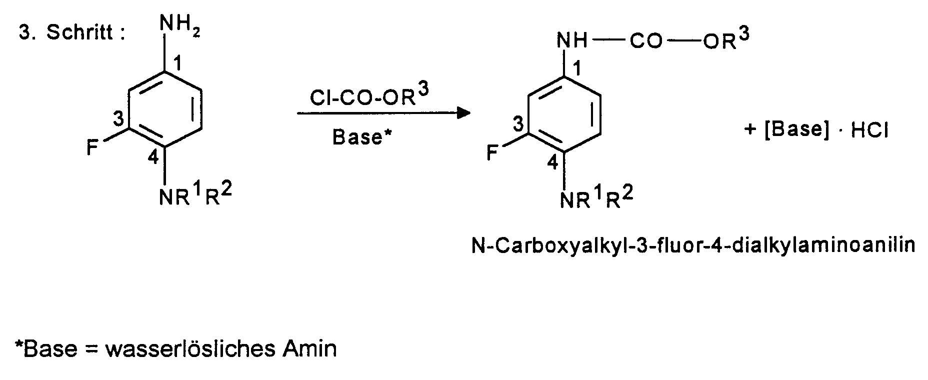

Wie zuvor bereits erwähnt, wird in einem ersten Schritt das Fluoratom der 4-Position im 2-Chlor-4,5-difluornitrobenzol durch Umsetzung mit dem sekundären Amin der Formel (II) in Gegenwart eines wasserlöslichen Amins, das bei Reaktionstemperatur und Aufarbeitungstemperatur flüssige oder im Reaktionsgemisch lösliche Hydrofluoride und Hydrochloride bildet, als Base in Anwesenheit oder Abwesenheit eines Lösungsmittels gegen einen Aminrest -NR¹R² ausgetauscht.

Man setzt als sekundäres Amin der Formel (II) Dimethylamin, Diethylamin, Di-n-propylamin, Di-i-propylamin, Di-n-butylamin, Di-i-butylamin, Piperidin, Morpholin oder Piperazin, insbesondere Piperidin, Morpholin oder Piperazin, bevorzugt Morpholin oder Piperazin ein.

Piperidin, Morpholin und Piperazin sind Beispiele für sekundäre Amine der Formel (II), worin R¹ und R² zusammen mit dem N-Atom, an dem sie stehen, einen Ring bilden. Dieser Ring weist jeweils 6 Glieder auf.

Bei der Umsetzung von 2-Chlor-4,5-difluornitrobenzol im ersten Schritt dient das sekundäre Amin lediglich als Reaktionspartner und als basische Verbindung wird ein wasserlösliches Amin zugesetzt, das insbesondere in organischer Phase lösliche (flüssige) Hydrofluoride und Hydrochloride bildet.

Man kann als wasserlösliches Amin ein tertiäres wasserlösliches Amin, das unter den Bedingungen der Reaktion und Aufarbeitung, insbesondere bei Reaktionstemperatur und Aufarbeitungstemperatur, flüssige oder im Reaktionsgemisch lösliche Hydrohalogenide, insbesondere Hydrofluoride und Hydrochloride bildet, verwenden. Derartige tertiäre wasserlösliche Amine finden sich in der Gruppe der Alkoxypolyoxyalkylamine.

Man kann mit guten Erfolg als wasserlösliches Amin ein Amin der Formel (III) NR³R⁴R⁵ einsetzen, worin R³, R⁴, R⁵ gleich oder verschieden sind, für einen geradkettigen oder verzweigten Alkylrest mit 1 bis 16, insbesondere 1 bis 8, bevorzugt 1 bis 4, besonders bevorzugt 1 bis 2 Kohlenstoffatomen oder einen Alkoxypolyoxyalkylrest -(CₘH₂ₘO)ₚR', worin R' ein Alkylrest mit 1 bis 16, insbesondere 1 bis 8, bevorzugt 1 bis 4, besonders bevorzugt 1 bis 2 Kohlenstoffatomen, m eine ganze Zahl von 1 bis 10, insbesondere 1 bis 5, bevorzugt 1 bis 3, besonders bevorzugt 2 und p eine ganze Zahl von 1 bis 15, insbesondere 2 bis 10, bevorzugt 2 bis 5, besonders bevorzugt 3 bis 4 ist, stehen und wenigstens einer der Reste R³, R⁴, R⁵ für einen Alkoxypolyoxyalkylrest -(CₘH₂ₘO)ₚR' steht, worin R', m und p die vorstehend genannte Bedeutung haben.

In vielen Fällen kann man als wasserlösliches Amin ein Amin der Formel (III) NR³R⁴R⁵, worin R³, R⁴, R⁵ gleich oder verschieden sind und wenigstens zwei der Reste für einen Alkoxypolyoxyalkylrest -(CₘH₂ₘO)ₚR', insbesondere für gleiche Alkoxypolyoxyalkylreste -(CₘH₂ₘO)ₚR', stehen, einsetzen.

Man kann als wasserlösliches Amin insbesondere ein Amin der Formel (III) NR³R⁴R⁵, worin R³, R⁴, R⁵ gleich oder verschieden sind und für einen Alkoxypolyoxyalkylrest -(CₘH₂ₘO)ₚR' stehen, einsetzen.

In vielen Fällen kann man als wasserlösliches Amin bevorzugt ein Amin der Formel (III), worin R³, R⁴, R⁵ gleich sind und für einen Alkoxypolyoxyalkylrest -(CₘH₂ₘO)ₚR' stehen, einsetzen.

Beispiele für gut geeignete wasserlösliche Amine sind (Tri-(methyltetraethoxy)amin, Tri-(butyltetraethoxy)amin, Tri-(ethyltetraethoxy)amin und Di-(methyltetraethoxy)methylamin, insbesondere Tri-(methyltetraethoxy)amin und Di-(methyltetraethoxy)methylamin.

Man setzt als Base 50 bis 500, insbesondere 90 bis 200, bevorzugt 100 bis 130 Mol-% wasserlösliches Amin, bezogen auf Äquivalent abzuspaltendes Fluorid oder Chlorid ein. Dies gilt für den ersten Schritt, für den zweiten Schritt und auch für den dritten Schritt in den Reaktionsfolge.

Aufgabe des wasserlöslichen Amins ist es, den in der ersten Stufe gebildeten Fluorwasserstoff und den in der zweiten und nachfolgenden Stufe jeweils gebildeten Chlorwasserstoff zu binden.

Man kann bei der Umsetzung des 2-Chlor-4,5-difluornitrobenzols auf eine Isolierung des 2-Chlor-4-dialkylamino-5-fluornitrobenzols verzichten und das im ersten Schritt anfallende Reaktionsgemisch unmittelbar weiterverarbeiten. Diese Variante des erfindungsgemäßen Verfahren ist besonders vorteilhaft, da sie sich besonders einfach durchführen läßt.

Kann man bei der Umsetzung des 2-Chlor-4,5-difluornitrobenzols in Gegenwart oder Abwesenheit eines Lösungsmittels arbeiten. Besonders vorteilhaft kann die Lösungsmittelmenge minimiert oder auf den Einsatz eines Lösungsmittels ganz verzichtet werden, wenn das wasserlösliche Amin selbst als Lösungsmittel verwendet wird.

In einer Vielzahl von Fällen kann man sowohl im ersten Schritt als auch im zweiten Schritt der Verwendung eines Lösungsmittels den Vorzug geben. Zur Durchführung des Verfahrens eignet sich eine Vielzahl verschiedener Lösungsmittel. Hierunter fallen unpolare Lösungsmittel, aprotische Lösungsmittel, dipolar aprotische Lösungsmittel und polar aprotische Lösungsmittel.

Ohne Anspruch auf Vollständigkeit zu erheben, sei erwähnt, daß man als Lösungsmittel einen aliphatischen Kohlenwasserstoff mit 5 bis 25 Kohlenstoffatomen, einen aromatischen Kohlenwasserstoff mit 6 bis 12 Kohlenstoffatomen, einen aliphatischen Alkohol mit 1 bis 12 Kohlenstoffatomen, ein Polyalkylenglykol mit 2 bis 6 Kohlenstoffatomen je Alkylen, einen Dialkylether mit 2 bis 20 Kohlenstoffatomen je Alkylrest, ein Polyalkylenglykoldialkylether mit 1 bis 6 Kohlenstoffatomen je Alkylen, einen Ester, ein Carbonsäuredialkylamid, Dialkylsulfoxid, Dialkylsulfon, ein Imidazolidinon, ein Pyrolidon oder ein Gemisch derselben, einsetzen kann.

Mit gutem Erfolg kann man als Lösungsmittel Benzol, Toluol, ortho-Xylol, meta-Xylol, para-Xylol, ein technisches Gemisch isomerer Xylole, Ethylbenzol, Mesitylen, Methanol, Ethanol, n-Propanol, i-Propanol, n-Butanol, i-Butanol, Dimethylformamid, Diethylformamid, Dimethylacetamid, Diethylacetamid, Dimethylsulfoxid, Dimethylsulfon, Sulfolan, 1,3-Dimethylimidazolidin-2-on, N-Pyrolidon oder ein Gemisch derselben, insbesondere Toluol, ortho-Xylol, meta-Xylol, para-Xylol, ein technisches Gemisch isomerer Xylole, Dimethylformamid, Dimethylacetamid, 1,3-Dimethylimidazolidin-2-on, Essigsäurealkylester mit 1 bis 12 Kohlenstoffatomen oder ein Gemisch derselben, bevorzugt Toluol, ortho-Xylol, meta-Xylol, para-Xylol, ein technisches Gemisch isomerer Xylole, Essigsäuremethylester, Essigsäureethylester oder Essigsäurebutylester, einsetzen.

In einer Reihe von Fällen hat es sich im ersten Schritt der Reaktionsfolge bewährt, die Umsetzung der Verbindung der Formel (I) mit dem sekundären Amin der Formel (II) bei 0 bis 100°C durchzuführen.

Nach einer besonderen Variante versetzt man das im ersten Schritt gebildete Reaktionsgemisch mit einer Base und setzt das wasserlösliche Amin aus seinem Hydrofluorid frei. Man versetzt das im ersten Schritt gebildete Reaktionsgemisch insbesondere mit einer wäßrigen Lösung eines Alkalimetallhydroxids, Erdalkalimetallhydroxids oder Gemisches dieser Hydroxide, bevorzugt mit einer wäßrigen NaOH oder KOH Lösung als Base.

Üblicherweise setzt man 0,8 bis 1,5, insbesondere 0,95 bis 1,05 Äquivalente Base je Mol Hydrofluorid ein.

Diese Variante ist besonders interessant, da sie eine Reihe von Vorteilen bietet. Zum einen wird das in Form seines Hydrofluorids gebundene wasserlösliche Amin auf recht einfache Weise zurückgewonnen, wodurch zum anderen die einzusetzende Menge wasserlösliches Amin gering gehalten werden kann. Ferner entfällt die Abtrennung des Hydrofluorids und auf eine Abtrennung des wasserlöslichen Amins kann ebenfalls verzichtet werden, da das wasserlösliche Amin auch im zweiten Schritt der Reaktionsfolge anwesend ist.

Es ist allerdings auch möglich, auf den Zusatz der Base zu dem im ersten Schritt gebildeten Reaktionsgemisch zu verzichten, das gebildete Hydrofluorid und gegebenenfalls das wasserlösliche Amin abzutrennen und das Hydrofluorid und das wasserlösliche Amin separat aufzuarbeiten.

Man setzt das aus dem ersten Schritt entstammende 2-Chlor-4-dialkylamino-5-fluornitrobenzol enthaltende Reaktionsgemisch oder 2-Chlor-4-dialkylamino-5-fluornitrobenzol in isolierter Form in Anwesenheit oder Abwesenheit eines Lösungsmittels in den zweiten Schritt ein.

Dabei kann das aus dem ersten Schritt entstammende 2-Chlor-4-dialkylamino-5-fluornitrobenzol enthaltende Reaktionsgemisch unmittelbar, das heißt ohne zusätzliche Aufarbeitungsschritte in den zweiten Schritt eingesetzt werden.

Setzt man 2-Chlor-4-dialkylamino-5-fluornitrobenzol in isolierter Form in den zweiten Schritt ein, so verwendet man üblicherweise ein Lösungsmittel. Als Lösungsmittel kann eines der vorstehend genannten Lösungsmittel und/oder das wasserlösliche Amin dienen.

Im zweiten Schritt wird das 2-Chlor-4-dialkylamino-5-fluornitrobenzol in Gegenwart des bereits zuvor beschriebenen wasserlöslichen Amins als Base und eines Edelmetallkatalysators hydriert. Hierbei wird die Nitrogruppe in eine NH₂-Gruppe umgewandelt und zugleich das in ortho-Stellung zur Nitrogruppe stehende Chlor, unter Bildung von Chlorwasserstoff abgespalten. Aufgabe der Base ist es, den freigesetzten Chlorwasserstoff zu binden.

Man setzt üblicherweise in den zweiten Schritt als Base dasselbe wasserlösliche Amin, wie im ersten Schritt, nämlich das tertiäre wasserlösliche Amin ein, das bei Reaktionstemperatur und Aufarbeitungstemperatur flüssige oder im Reaktionsgemisch lösliche Hydrofluoride oder Hydrochloride bildet, ein.

Besonders geeignet als wasserlösliches Amin für den zweiten Schritt der Reaktionsfolge sind Tri-(methyltetraethoxy) amin, Tri-(butyltetramethoxy)amin, Tri-(ethyltetraethoxy)amin und Di-(methyltetraethoxy)-methylamin.

Man setzt als Base in dem zweiten Schritt 50 bis 500, insbesondere 90 bis 200, bevorzugt 100 bis 130 Mol-% wasserlösliches Amin, bezogen auf Äquivalent abzuspaltendes Chlorid, ein. Wurde das wasserlösliche Amin im vorangegangenen ersten Reaktionsschritt als Lösungsmittel mit entsprechendem molarem Überschuß verwendet und/oder aus dem gebildeten Hydrofluorid freigesetzt, kann auf eine weitere Zugabe im zweiten Reaktionsschritt verzichtet werden. Es muß lediglich sichergestellt sein, daß das in den zweiten Schritt gelangende Reaktionsgemisch respektive Produkt das wasserlösliche Amin bereits in ausreichender Menge enthält.

Setzt man 2-Chlor-4-dialkylamino-5-fluornitrobenzol in isolierter Form ein, so muß man das wasserlösliche Amin in der zuvor angegebenen Menge als Base zusetzen.

Die Base hat die Aufgabe, den während der dechlorierenden Hydrierung abgespaltenen Chlorwasserstoff zu binden.

Man setzt als Edelmetallkatalysator einen Palladiumträgerkatalysator ein. Der Edelmetallkatalysator enthält 0,1 bis 25, insbesondere 0,5 bis 10, bevorzugt 1,0 bis 5,0 Gew.-% Palladium.

Der Edelmetallkatalysator enthält Aktivkohle, Calciumcarbonat, Bariumsulfat, Bimsstein, Tonerde, Kieselgur, Kieselgel, Aluminiumoxid oder ein Gemisch derselben, insbesondere Aktivkkohle, Kieselgur, Aluminiumoxid oder ein Gemisch derselben, bevorzugt Aktivkohle als Trägermaterial.

Es hat sich besonders bewährt, einen Palladium-Aktivkohle-Katalysator als Edelmetallkatalysator einzusetzen.

Man führt die Hydrierung in Gegenwart von Wasserstoff durch, wobei man die Umsetzung in einer Vielzahl von Fällen bei einem Druck von 2 bis 100 bar, insbesondere bei einem Druck von 5 bis 50 bar und bei einer Temperatur von 40 bis 140°C, insbesondere 60 bis 130°C durchführt.

Bei Durchführung des zweiten Schritts der Reaktionsfolge ist darauf zu achten, daß man ein Lösungsmittel verwendet, das unter den Bedingungen der Hydrierung inert ist. Ungeeignet als Lösungsmittel sind chlorierte aliphatische oder aromatische Kohlenwasserstoffe, da diese unter den Reaktionsbedingungen mit Wasserstoff reagieren können. Diese Einschränkungen hinsichtlich des Lösungsmittels gilt lediglich für die Stufe der Hydrierung. Um einen Wechsel des Lösungsmittels zu vermeiden, ist es vorteilhaft, bereits in den ersten Schritt der Reaktionsfolge ein Lösungsmittel, das auch für den zweiten Schritt der Reaktionsfolge geeignet ist, einzusetzen.

Geeignete Lösungsmittel sind beispielsweise die bereits vorstehend genannten Solventien.

Nach einer besonderen Variante versetzt man das im zweiten Schritt gebildete Reaktionsgemisch, gegebenenfalls nach Abtrennung des Edelmetallkatalysators mit einer Base und setzt das wasserlösliche Amin aus seinem Hydrochlorid frei. Man versetzt das im zweiten Reaktionsschritt gebildete Reaktionsgemisch insbesondere mit einer wäßrigen Lösung eines Alkalimetallhydroxids, Erdalkalimetallhydroxids oder Gemisches dieser Hydroxide, bevorzugt mit einer wäßrigen NaOH oder KOH Lösung als Base.

Üblicherweise setzt man 0,8 bis 1,5, insbesondere 0,95 bis 1,05 Äquivalente Base je Mol Hydrochlorid ein.

Diese Variante ist besonders interessant, da sie eine Reihe von Vorteilen bietet. Zum einen wird das in Form eines Hydrochlorids gebundene wasserlösliche Amin auf recht einfache Weise zurückgewonnen, wodurch zum anderen die einzusetzende Menge wasserlösliches Amin gering gehalten werden kann. Ferner entfällt die Abtrennung des Hydrochlorids und auf eine Abtrennung des wasserlöslichen Amins kann ebenfalls verzichtet werden, da das wasserlösliche Amin auch im dritten Schritt der Reaktionsfolge anwesend ist.

Es ist allerdings auch möglich, auf den Zusatz der Base zu dem im zweiten Schritt gebildeten Reaktionsgemisch zu verzichten, das gebildete Hydrochlorid und gegebenenfalls das wasserlösliche Amin abzutrennen und das Hydrochlorid und das wasserlösliche Amin separat aufzuarbeiten.

Man trennt üblicherweise den Edelmetallkatalysator von dem Reaktionsgemisch, beispielsweise durch Filtration, Sedimentation oder Zentrifugieren, ab. Die Abtrennung kann vor dem Zusatz der Base oder danach erfolgen. Ist der Edelmetallkatalysator als Festbett angeordnet, so ist eine separate Abtrennung des Katalysators nicht erforderlich, da der Katalysator im Festbett in stückiger Form angeordnet verbleibt und nicht in den Produktstrom gelangt.

Das aus dem zweiten Schritt resultierende, das entsprechende 3-Fluor-4-dialkylaminoanilin enthaltende Reaktionsgemisch wird in einem dritten Schritt in Anwesenheit des wasserlöslichen Amins mit einem Chlorameisensäureester der Formel (IV) Cl-CO-OR³ unter Bildung des entsprechenden N-Carboxyalkyl-3-fluordiaminoanilins umgesetzt.

Aufgabe des wasserlöslichen Amins ist es, den im dritten Schritt gebildeten Chlorwasserstoff zu binden.

Man setzt als Base im dritten Schritt 50 bis 500, insbesondere 90 bis 200, bevorzugt 100 bis 130 Mol.-% wasserlösliches Amin, bezogen auf Äquivalent abzuspaltendes Fluorid oder Chlorid, ein.

Wurde das wasserlösliche Amin im vorangegangenen zweiten Reaktionschritt als Lösungsmittel mit entsprechenden molaren Überschuß verwendet und/oder aus dem gebildeten Hydrochlorid freigesetzt, kann auf eine weitere Zugabe im dritten Schritt der Reaktionsfolge verzichtet werden.

Wenn das verwendete wasserlösliche Amin nach der zweiten Stufe bereits aufgebraucht ist, kann auf eine weitere Zugabe verzichtet werden, wenn das verbrauchte wasserlösliche Amin durch die Zugabe einer Alkali- oder Erdalkalibase wieder regeneriert wird. Dafür muß lediglich die benötigte molare Menge an Base, beispielsweise Natronlauge zugesetzt werden. Gegebenenfalls kann die in Abhängigkeit der Wasserkonzentration der Reaktionslösung sich bildende wäßrige Phase abgetrennt werden. Die weitere Umsetzung gelingt allerdings auch in Gegenwart einer wäßrigen Phase.

Es muß lediglich sichergestellt sein, das das in den dritten Schritt gelangende Reaktionsgemisch das wasserlösliche Amin bereits in ausreichender Menge enthält.

Man setzt als Chlorameisensäureester der Formel (IV) insbesondere eine Verbindung ClCO₂R³, worin R³ ein Alkylrest mit 1 bis 8, insbesondere 1 bis 4 Kohlenstoffatomen oder ein Benzylrest ist, ein.

Für die dritte Stufe eignen sich als Chlorameisensäureester beispielsweise Chlorameisensäureemethylester, Chlorameisensäureethylester, Chlorameisensäure-n-propylester, Chlorameisensäure-i-propyl-ester, Chlorameisensäure-n-butylester, Chlorameisensäure-i-butylester, Chlorameisensäurebenzylester, bevorzugt Chlorameisensäurebenzylester.

In den meisten Fällen erweist es sich als ausreichend, die Umsetzung mit dem Chlorameisensäureester bei 10 bis 80°C durchzuführen.

Nach einer besonderen Variante versetzt man das im dritten Schritt gebildete, das entsprechende N-Carboxyalkyl-3-fluor-4-dialkylaminoanilin enthaltende Reaktionsgemisch mit einer Base und setzt das wasserlösliche Amin aus seinem Hydrochlorid frei. Man versetzt das im dritten Schritt gebildete Reaktionsgemisch insbesondere mit einer wäßrigen Lösung eines Alkalimetallhydroxids, Erdalkalimetallhydroxids oder Gemisches dieser Hydroxide, bevorzugt mit einer wäßrigen NaOH oder KOH Lösung als Base.

Üblicherweise setzt man 0,9 bis 2, insbesondere 1 bis 1,1 Äquivalente Base je Mol Hydrochlorid ein.

Diese Variante ist besonders interessant, da sie eine Reihe von Vorteilen bietet. Zum einen wird das in Form seines Hydrochlorids gebundene wasserlösliche Amin auf recht einfache Weise zurückgewonnen und kann nach Abschluß der Synthese, gegebenenfalls nach Aufarbeitung, wieder in den ersten Schritt eingesetzt werden, wodurch zum anderen die einzusetzende Menge Amin gering gehalten werden kann und nur durch geringe Mengen frischen wasserlöslichen Amins zu ergänzen ist. Ferner entfällt eine Abtrennung des Hydrochlorids. Lediglich die durch die Freisetzung des wasserlöslichen Amins aus seinen Hydrofluoriden und Hydrochloriden gebildeten Salze (Fluoride im ersten Schritt, Chloride im zweiten und dritten Schritt) müssen abgetrennt werden.

Die Salze (Fluoride und Chloride) fallen gegebenenfalls durch Zusatz von Wasser, ebenso wie das wasserlösliche Amin als eine wäßrige Lösung an, aus der sich das Wertprodukt, nämlich das entsprechende N-Carboxyalkyl-3-fluor-4-dialkylaminoanilin, üblicherweise als Feststoff abscheidet. Man filtriert das Wertprodukt ab und kristallisiert es zur weiteren Reinigung um. Aus der wäßrigen Lösung läßt sich das wasserlösliche Amin und gegebenenfalls Lösungsmittel zurückgewonnen.

Arbeitet man in Anwesenheit einer wasserunlöslichen Lösungsmittels, so fällt das Reaktionsgemisch in Lösung an und kann von der wäßrigen Phase durch einfache Phasenseparation abgetrennt werden.

Nach erfolgter Umsetzung erhält man das N-Carboxyalkyl-3-fluor-4-dialkylaminoanilin üblicherweise in der Lösung aller bis dahin zugegebenen Reaktions- und Hilfsstoffe. Das gebildete N-Carboxyalkyl-3-fluor-4-dialkylaminoanilin kann durch Eintropfen in Wasser als Feststoff ausgefällt werden, das durch Filtration und/oder Extraktion mittels eines organischen, in Wasser unlöslichen Solvens abgetrennt und isoliert werden kann. Das als Base verwendete Amin löst sich dabei im Wasser und kann nach der Regenerierung (z.B. Freisetzung durch NaOH) und der Abtrennung des Wasser in den Folgeansatz zurückgeführt werden.

Falls gewünscht, kann das Wertprodukt noch einer weiteren Reinigung, beispielsweise durch Kristallisation zugeführt werden.

Als besondere Variante des Verfahrens kann nach dem ersten, zweiten und dritten Schritt jeweils die verbrauchte Menge Amin durch die Zugabe von Natronlauge wieder zurückgewonnen werden, so daß die benötigte Menge an Amin für die Reaktion auf ein Minimum von 1 bis 2 Äquivalenten wasserlösliches Amin pro Mol Edukt reduziert werden kann.

Das erfindungsgemäße Verfahren läßt sich unter reduziertem Druck, Atmosphärendruck oder erhöhtem Druck durchführen.

Die Erfindung betrifft ferner die Verbindungen N-Carboxymethyl-3-fluor-4-morpholinoanilin und N-Carboxyisobutyl-3-fluor-4-morpholinoanilin.

### Experimenteller Teil

### Beispiel 1: Herstellung von N-Carboxybenzyl-3-fluor-4-morpholinoanilin

In einem 2000 ml Autoklaven mit Begasungsrührer werden bei 50°C in eine Lösung von 135,5 g (0,7 mol) 2-Chlor-4,5-difluornitrobenzol in 1192 g (2,0 mol) Tri-(methyltetraethoxy)-amin innerhalb einer Stunde 67,1 g (0,77 mol) Morpholin eingetragen. Anschließend wird für 3 Stunden bei gleicher Temperatur nachgerührt, 21 g eines Palladium-Aktivkohle-Katalysators (5 Gew.-% Palladium, 50% wasserfeucht) zudosiert, der Autoklav geschlossen und mit Stickstoff inertisiert. Die Reaktionstemperatur wird auf 105°C gesteigert und bei dieser Temperatur bei einem Wasserstoffdruck von 5 bis 30 bar solange gehalten, bis keine Wasserstoffaufnahme mehr erfolgt. Nach dem Absaugen des Katalysators wird die Reaktionslösung bei Raumtemperatur mit 149,3 g (0,88 mol) Chlorameisensäurebenzylester (95%-ig) versetzt. Nach beendeter Zugabe wird noch eine Stunde nachgerührt, das Lösungsmittel im Vakuum entfernt und der verbleibende Rückstand in 1000 ml Wasser eingetragen. Der gebildete Feststoff wird abgesaugt, mit Wasser gewaschen und anschließend aus Methanol umkristallisiert.

Aus dem verbleibenden Abwasser kann anschließend durch Zugabe von Natronlauge das Amin regeneriert werden.

Alle Reaktionsschritte werden unter Schutzgas durchgeführt.

### Ausbeute:

173,3 g (0,53 mol) N-Carboxybenzyl-3-fluor-4-morpholinoanilin, das entspricht einer theoretischen Ausbeute von 75 %, bezogen auf eingesetztes 2-Chlor-4,5-difluornitrobenzol

### Beispiel 2: Herstellung von N-Carboxybenzyl-3-fluor-4-morpholinoanilin

In einem 2000 ml Autoklaven mit Begasungsrührer wird bei 110°C eine Lösung von 156,3 g (0,6 mol) 2-Chlor-5-fluor-4-morpholinonitrobenzol in 763 g (1,3 mol) Tri-(methyltetraethoxy)-amin und 260 g Toluol zusammen mit 18 g eines Palladium-Aktivkohle-Katalysators (5 Gew.-% Palladium, 50% wasserfeucht) vorgelegt. Der Autoklav wird geschlossen, mit Stickstoff inertisiert und bei einer Reaktionstemperatur von 95 bis 110°C mit Wasserstoff bei einem Druck von 5 bis 30 bar solange umgesetzt, bis keine Wasserstoffaufnahme mehr erfolgt. Nach dem Absaugen des Katalysators wird die Reaktionslösung bei Raumtemperatur mit 128 g (0,71 mol) Chlorameisensäurebenzylester (95%-ig) versetzt. Nach beendeter Zugabe wird noch eine Stunde nachgerührt, das Lösungsmittel im Vakuum entfernt und der verbleibende Rückstand in 1000 ml Wasser eingetragen. Der gebildete Feststoff wird abgesaugt, mit Wasser gewaschen und anschließend aus Methanol umkristallisiert. Aus dem verbleibenden Abwasser kann anschließend durch Zugabe von Natronlauge das Amin regeneriert werden.

### Alle Reaktionsschritte werden unter Schutzgas durchgeführt.

### Ausbeute:

156,4 g (0,47 mol) N-Carboxybenzyl-3-fluor-4-morpholinoanilin, das entspricht einer theoretischen Ausbeute von 79 %, bezogen auf eingesetztes 2-Chlor-5-fluor-4-morpholinonitrobenzol

### Beispiel 3: Herstellung von N-Carboxybenzyl-3-fluor-4-morpholinoanilin

In einem 2000 ml Autoklaven mit Begasungsrührer wird bei 110°C eine Lösung von 208,4 g (0,8 mol) 2-Chlor-5-fluor-4-morpholinonitrobenzol in 611 g (1,04 mol) Tri-(methyltetraethoxy)-amin und 400 g Essigsäurebutylester zusammen mit 18 g eines Palladium-Aktivkohle-Katalysators (5 Gew.-% Palladium, 50% wasserfeucht) vorgelegt.

Der Autoklav wird geschlossen, mit Stickstoff inertisiert und bei einer Reaktionstemperatur von 95 bis 110°C mit Wasserstoff bei einem Druck von 5 bis 30 bar solange umgesetzt, bis keine Wasserstoffaufnahme mehr erfolgt. Nach dem Absaugen des Katalysators wird die Reaktionslösung bei Raumtemperatur mit 57,5 g (0,72 mol) NaOH 50%-ig versetzt und anschließend werden 161,6 g (0,9 mol) Chlorameisensäurebenzylester (95%-ig) zudosiert. Nach beendeter Zugabe wird noch eine Stunde bei 50°C nachgerührt, mit weiteren 72 g (0,9 mol) NaOH 50%-ig und 150 g Wasser versetzt und die sich bildende wäßrige Phase abgetrennt. Anschließend wird das Lösungsmittel im Vakuum entfernt und der verbleibende Rückstand in 800 ml Wasser eingetragen. Der gebildete Feststoff wird abgesaugt, mit Wasser gewaschen und anschließend aus Methanol umkristallisiert.

Aus dem verbleibenden Abwasser kann anschließend durch Zugabe von Natronlauge das Amin regeneriert werden.

Alle Reaktionsschritte werden unter Schutzgas durchgeführt.

### Ausbeute:

221,8 g (0,67 mol) N-Carboxybenzyl-3-fluor-4-morpholinoanilin, das entspricht einer theoretischen Ausbeute von 84 %, bezogen auf eingesetztes 2-Chlor-5-fluor-4-morpholinonitrobenzol

### Beispiel 4: Herstellung von N-Carboxybenzyl-3-fluor-4-morpholinoanilin

In einem 2000 ml Autoklaven mit Begasungsrührer wird bei Raumtemperatur eine die Suspension von 208,4 g (0,8 mol) 2-Chlor-5-fluor-4-morpholinonitrobenzol in 516,6 g (0,88 mol) Tri-(methyltetraethoxy)-amin und 105 g Methanol zusammen mit 18 g eines Palladium-Aktivkohle-Katalysators (5 Gew.-% Palladium, 50% wasserfeucht) vorgelegt. Der Autoklav wird geschlossen, mit Stickstoff inertisiert und bei einer Reaktionstemperatur von 80 bis 100°C mit Wasserstoff bei einem Druck von 5 bis 30 bar solange umgesetzt, bis keine weitere Wasserstoffaufnahme mehr erfolgt. Nach dem Absaugen des Katalysators wird die Reaktionslösung bei Raumtemperatur mit 57,5 g (0,72 mol) NaOH 50%-ig versetzt und anschließend werden 161,6 g (0,9 mol) Chlorameisensäurebenzylester (95%-ig) zudosiert. Nach beendeter Zugabe wird noch 15 Minuten bei 50°C nachgerührt und die Reaktionslösung/suspension mit 775 g Wasser und 36 g (0,9 mol) NaOH versetzt. Der gebildete Feststoff wird abgesaugt, mit Wasser gewaschen und anschließend aus Methanol umkristallisiert.

Aus dem verbleibenden Abwasser kann anschließend durch Zugabe von äquimolaren Mengen an Natronlauge Tri-(methyltetraethoxy)-amin und Methanol regeneriert werden.

Alle Reaktionsschritte werden unter Schutzgas durchgeführt.

### Ausbeute:

208,0 g (0,63 mol) N-Carboxybenzyl-3-fluor-4-morpholinoanin, das entspricht einer theoretischen Ausbeute von 78,8 %, bezogen auf eingesetztes eingesetztes 2-Chlor-5-fluor-4-morpholinonitrobenzol.

### Beispiel 5: Herstellung von N-Carboxymethyl-3-fluor-4-morpholinoanilin

In einem 2000 ml Autoklaven mit Begasungsrührer wird bei Raumtemperatur eine Suspension von 208,4 g (0,8 mol) 2-Chlor-5-fluor-4-morpholinonitrobenzol in 516,6 g (0,88 mol) Tri-(methyltetraethoxy)-amin und 105 g Methanol zusammen mit 18 g eines Palladium-Aktivkohle-Katalysators (5 Gew.-% Palladium, 50% wasserfeucht) vorgelegt. Der Autoklav wird geschlossen, mit Stickstoff inertisiert und bei einer Reaktionstemperatur von 80 bis 100°C mit Wasserstoff bei einem Druck von 5 bis 30 bar solange umgesetzt, bis keine Wasserstoffaufnahme mehr erfolgt. Nach dem Absaugen des Katalysators wird die Reaktionslösung bei Raumtemperatur mit 57,5 g (0,72 mol) NaOH 50%-ig versetzt und anschließend werden 94,5 g (1,0 mol) Chlorameisensäuremethylester (97%-ig) zudosiert. Nach beendeter Zugabe wird noch 15 Minuten bei 50°C nachgerührt und die Reaktionslösung/suspension mit 775 g Wasser und 36 g (0,9 mol) NaOH versetzt. Der gebildete Feststoff wird abgesaugt, mit Wasser gewaschen und anschließend aus Methanol umkristallisiert.

Aus dem verbleibenden Abwasser kann anschließend durch Zugabe von äquimolaren Mengen an Natronlauge Tri-(methyltetraethoxy)-amin und Methanol regeneriert werden.

Alle Reaktionsschritte werden unter Schutzgas durchgeführt.

### Ausbeute:

150,0 g (0,59 mol) N-Carboxymethyl-3-fluor-4-morpholinoanilin, das entspricht einer theoretischen Ausbeute von 73,8 % bezogen auf eingesetztes 2-Chlor-5-fluor-4-morpholinonitrobenzol.

Schmelzpunkt: 163,5 °C
¹H-NMR: δ (TMS) = 0, (CDCl₃): δ = 3,86, 3,76, 3,03, 6,74 (NH), 6,86 (J_{F,H} = 9,0 Hz); 6,98; 7,27 (J_{F,H} = 13,0 Hz)
¹³C-NMR: δ (CDCl₃) = 77, (CDCl₃): δ = 51,18 (J_{F,C} = 3,0 Hz); 52,34; 66,99; 108,02 (J_{F,C} = 25 Hz); 114,65 (J_{F,C} = 0 Hz); 119,04 (J_{F,C} = 4,2 Hz); 133,25 (J_{F,C} = 10,7 Hz); 135,87 (J_{F,C} = 9,0 Hz); 154,03 (J_{F,C} = 0 Hz); 155,71 (J_{F,C} = 245,8 Hz)

### Beispiel 6: Herstellung von N-Carboxy-i-butyl-3-fluor-4-morpholinoanilin

In einem 2000 ml Autoklaven mit Begasungsrührer wird bei Raumtemperatur in die Suspension von 208,4 g (0,74 mol) 2-Chlor-5-fluor-4-morpholinonitrobenzol in 477,9 g (0,81 mol) Tri-(methyltetraethoxy)-amin und 97,1 g Methanol zusammen mit 16,7 g eines Palladium-Aktivkohle-Katalysators (5 Gew.-% Palladium, 50% wasserfeucht) vorgelegt. Der Autoklav wird geschlossen, mit Stickstoff inertisiert und bei einer Reaktionstemperatur von 80 bis 100°C mit Wasserstoff bei einem Druck von 5 bis 30 bar solange umgesetzt, bis keine Wasserstoffaufnahme mehr erfolgt. Nach dem Absaugen des Katalysators wird die Reaktionslösung bei Raumtemperatur mit 54,0 g (0,67 mol) NaOH 50%-ig versetzt und anschließend 114,4 g (0,8 mol) Chlorameisensäure-i-butylester (98%-ig) zudosiert. Nach beendeter Zugabe wird noch 15 Minuten bei 50°C nachgerührt und die Reaktionslösung/suspension mit 775 g Wasser und 36 g (0,9 mol) NaOH versetzt. Der gebildete Feststoff wird abgesaugt, mit Wasser gewaschen und anschließend aus Methanol umkristallisiert.

Aus dem verbleibenden Abwasser kann anschließend durch Zugabe von äquimolaren Mengen an Natronlauge Tri-(methyltetraethoxy)-amin und Methanol regeneriert werden.

Alle Reaktionsschritte werden unter Schutzgas durchgeführt.

### Ausbeute:

159,2 g (0,54 mol) N-Carboxy-i-butyl-3-fluor-4-morpholinoanilin, das entspricht einer theoretischen Ausbeute von 72,7 % bezogen auf eingesetztes 2-Chlor-5-fluor-4-morpholinonitrobenzol

Schmelzpunkt: 121,0 °C
¹H-NMR: δ (TMS) = 0, (CDCl₃): δ = 0,96; 1,97; 3,03; 3,86; 3,94; 6,64 (NH), 6,87 (J_{F,H} = 9,0 Hz); 6,98; 7,28 (J_{F,H} = 13,0 Hz)
¹³C-NMR: δ (CDCl₃) = 77, (CDCl₃): δ = 19,0; 27,96; 51,21 (J_{F,C} = 3,0 Hz); 67,01; 71,48; 107,96 (J_{F,C} = 25 Hz); 114,53 (J_{F,C} = 0 Hz); 119,06 (J_{F,C} = 4,2 Hz); 133,41 (J_{F,C} = 11,2 Hz); 135,73 (J_{F,C} = 9,4 Hz); 153,71 (J_{F,C} = 0 Hz); 155,75 (J_{F,C} = 245,9 Hz)

## Patentansprüche

1. Verfahren zur Herstellung von N-Carboxyalkyl-3-fluor-4-dialkylaminoanilinen, dadurch gekennzeichnet, daß man in einem ersten Schritt 2-Chlor-4,5-difluornitrobenzol der Formel (I) 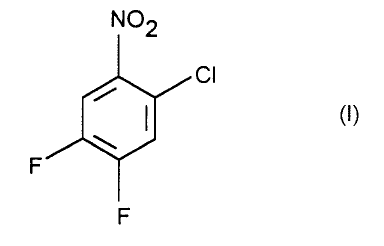 mit einem sekundären Amin der Formel (II) HNR¹R², worin R¹ und R² unabhängig voneinander gleich oder verschieden sind und für einen Alkylrest mit 1 bis 10 Kohlenstoffatomen stehen oder zusammen mit dem N-Atom, an dem sie stehen, einen Ring mit 5 bis 7 Gliedern bilden, in Gegenwart eines wasserlöslichen Amins, das bei Reaktionstemperatur und Aufarbeitungstemperatur flüssige oder im Reaktionsgemisch lösliche Hydrofluoride und Hydrochloride bildet, in Anwesenheit oder Abwesenheit eines Lösungsmittels bei -10 bis 120°C umsetzt, in einem zweiten Schritt das 2-Chlor-4-dialkylamino-5-fluornitrobenzol enthaltende Reaktionsgemisch in Anwesenheit des wasserlöslichen Amins in Gegenwart eines Edelmetallkatalysators mit Wasserstoff bei 30 bis 150°C und 1 bis 350 bar reduziert und in einem dritten Schritt das 3-Fluor-4-dialkylaminoanilin enthaltende Reaktionsgemisch in Anwesenheit des wasserlöslichen Amins mit einem Chlorameisensäureester der Formel (IV) ClCO₂R³, worin R³ für einen Alkylrest mit 1 bis 10 Kohlenstoffatomen oder Aralkylrest mit 7 bis 20 Kohlenstoffatomen steht, bei 0 bis 100°C umsetzt.

2. Verfahren nach Anspruch 1, dadurch gekennzeichnet, daß man als sekundäres Amin der Formel (II) Dimethylamin, Diethylamin, Di-n-propylamin, Di-i-propylamin, Di-n-butylamin, Di-i-butylamin, Piperidin, Morpholin oder Piperazin einsetzt.

3. Verfahren nach Anspruch 1 oder 2, dadurch gekennzeichnet, daß man als wasserlösliches Amin ein Amin der Formel (III) NR⁴R⁵R⁶, worin R⁴, R⁵, R⁶ gleich oder verschieden sind, für einen Alkylrest mit 1 bis 16 Kohlenstoffatomen oder einen Alkoxypolyoxyalkylrest -(CₘH₂ₘO)ₚR', worin R' ein Alkylrest mit 1 bis 16 Kohlenstoffatomen, m eine ganze Zahl von 1 bis 10 und p eine ganze Zahl von 1 bis 15 ist, stehen und wenigstens einer der Reste R⁴, R⁵, R⁶ ein Alkoxypolyoxyalkylrest -(CₘH₂ₘO)ₚR' ist, einsetzt.

4. Verfahren nach einem oder mehreren der Ansprüche 1 bis 3, dadurch gekennzeichnet, daß man als wasserlösliches Amin Tri-(methyltetraethoxy)-amin, Tri-(butyltetrethoxy)amin, Tri-(ethyltetraethoxy)amin oder Di-(methyltetraethoxy)methylamin einsetzt.

5. Verfahren nach einem oder mehreren der Ansprüche 1 bis 4, dadurch gekennzeichnet, daß man in den ersten Schritt 50 bis 500 Mol-% wasserlösliches Amin, bezogen auf Äquivalent abzuspaltendes Fluorid, einsetzt.

6. Verfahren nach einem oder mehreren der Ansprüche 1 bis 5, dadurch gekennzeichnet, daß man als Lösungsmittel einen aliphatischen Kohlenwasserstoff mit 5 bis 25 Kohlenstoffatomen, einen aromatischen Kohlenwasserstoff mit 6 bis 12 Kohlenstoffatomen, einen aliphatischen Alkohol mit 1 bis 12 Kohlenstoffatomen, ein Polyalkylenglykol mit 2 bis 6 Kohlenstoffatomen je Alkylen, einen Dialkylether mit 2 bis 20 Kohlenstoffatomen je Alkylrest, einen Polyalkylenglykoldialkylether mit 1 bis 6 Kohlenstoffatomen je Alkylen, einen Ester, ein Carbonsäuredialkylamid, ein Nitril, ein Dialkylsulfoxid, ein Dialkylsulfon, ein Imidazolinon, ein Pyrrolidon oder ein Gemisch derselben einsetzt.

7. Verfahren nach einem oder mehreren der Ansprüche 1 bis 6, dadurch gekennzeichnet, daß man das im ersten Schritt gebildete Reaktionsgemisch mit einer Base versetzt und das wasserlösliche Amin aus seinem Hydrofluorid freisetzt.

8. Verfahren nach einem oder mehreren der Ansprüche 1 bis 7, dadurch gekennzeichnet, daß man das im ersten Schritt gebildete Reaktionsgemisch mit einer wäßrigen Lösung eines Alkalimetallhydroxids, Erdalkalimetallhydroxids oder Gemisches dieser Hydroxide als Base versetzt,

9. Verfahren nach einem oder mehreren der Ansprüche 1 bis 8, dadurch gekennzeichnet, daß man das aus dem ersten Schritt entstammende 2-Chlor-4-dialkylamino-5-fluornitrobenzol enthaltende Reaktionsgemisch oder 2-Chlor-4-dialkylamino-5-fluornitrobenzol in isolierter Form in Anwesenheit oder Abwesenheit eines Lösungsmittels in den zweiten Schritt einsetzt.

10. Verfahren nach einem oder mehreren der Ansprüche 1 bis 9, dadurch gekennzeichnet, daß man in den zweiten Schritt 50 bis 500 Mol-% wasserlösliches Amin, bezogen auf Äquivalent abzuspaltendes Chlorid, einsetzt.

11. Verfahren nach einem oder mehreren der Ansprüche 1 bis 10, dadurch gekennzeichnet, daß man als Edelmetallkatalysator einen Palladium-Trägerkatalysator einsetzt.

12. Verfahren nach einem oder mehreren der Ansprüche 1 bis 11, dadurch gekennzeichnet, daß man das im zweiten Schritt gebildete Reaktionsgemisch, gegebenenfalls nach Abtrennung des Edelmetallkatalysators, mit einer Base versetzt und das wasserlösliche Amin aus seinem Hydrochlorid freisetzt.

13. Verfahren nach einem oder mehreren der Ansprüche 1 bis 12, dadurch gekennzeichnet, daß man in den dritten Schritt 50 bis 500 Mol-% wasserlösliches Amin, bezogen auf Äquivalent abzuspaltendes Chlorid, einsetzt.

14. Verfahren nach einem oder mehreren der Ansprüche 1 bis 13, dadurch gekennzeichnet, daß man als Chlorameisensäureester der Formel (IV) ClCO₂R³, worin R³ ein Alkylrest mit 1 bis 8 Kohlenstoffatomen oder ein Benzylrest ist, einsetzt.

15. Verfahren nach einem oder mehreren der Ansprüche 1 bis 14, dadurch gekennzeichnet, daß man das im dritten Schritt gebildete Reaktionsgemisch mit einer Base versetzt und das wasserlösliche Amin aus seinem Hydrochlorid freisetzt.

16. Die Verbindungen N-Carboxymethyl-3-fluor-4-morpholinoanilin und N-Carboxyisobutyl-3-fluor-4-morpholinoanilin.
